# EUROPEAN PATENT APPLICATION

(11) **EP 3 305 204 A1**
(43) Date of publication of application: **11.04.2018**
(21) Application number: 16803468.4
(22) Date of filing: 02.06.2016
(51) Int. Cl.: A61B 8/12, A61B 1/00

(54) **ULTRASONIC ENDOSCOPE AND WATER POURING DEVICE**

(30) Priority: 04.06.2015 JP 2015114188
(71) Applicant: Olympus Corporation, Hachioji-shi, Tokyo 192-8507 (JP)
(72) Inventor: TANIGUCHI, Yuko, Hachioji-shi Tokyo 192-8507 (JP); KODAMA, Hiroshi, Hachioji-shi Tokyo 192-8507 (JP)
(74) Representative: Gunzelmann, Rainer
(86) International application number: PCT/JP2016/066459
(87) International publication number: WO 2016/195034

(57) **Abstract**

An ultrasound endoscope according to the present invention includes: an insertion portion (10) configured to be inserted into a living body; a distal end rigid portion (10a) which is provided at a distal end of the insertion portion (10), includes an ultrasound transmitting and receiving unit (101), includes groove portions (102, 103) to which a balloon (106) surrounding the ultrasound transmitting and receiving unit (101) is mounted, and in which a balloon water supply port (104) configured to supply water to the balloon (106) is formed; a balloon water supply channel (128) communicating with the balloon water supply port (104); a water supply pipe (124) which is provided in a universal cable (12) that connects the connector portion (13) connecting the ultrasound endoscope (2) to a light source device (6) and an operating unit (11); an air supply and water supply cylinder (121) which is provided in the operating unit (11) and to which one end of the balloon water supply channel (128) and one end of the water supply pipe (124) are opened; and a duct (136) to inject water into an injection port (135) provided in the balloon water supply channel (128).

## Description

### Field

The present invention relates to an ultrasound endoscope for observing inside of a living body using ultrasound and to an injection tool provided in the ultrasound endoscope.

### Background

Ultrasound endoscopes are used by covering an ultrasound transmitting and receiving unit using a balloon and filling the balloon with water in order to achieve acoustic matching between the ultrasound transmitting and receiving unit and an examination target (such as an organ) (for example, see Patent Literature 1). The balloon has a structure in which an O-ring is provided at an aperture of a flexible film (rubber film) formed in a tubular shape or a bag shape, and is attached to the ultrasound transmitting and receiving unit by fitting the O-ring into a groove for engagement provided in the ultrasound transmitting and receiving unit.

### Citation List

### Patent Literature

Patent Literature 1: JP 2006-280407 A

### Summary

### Technical Problem

The balloon is fitted into the groove for engagement in close contact with the groove in order to prevent leakage of water filled inside the balloon. Thus, work at the time of removing the balloon is extremely difficult after using the ultrasound endoscope. In particular, the entire balloon is stuck to the ultrasound transmitting and receiving unit in a state where the balloon deflates, and the removal work becomes further difficult.

There is a case where the work of removing the balloon is performed using an instrument such as tweezers. In this case, a fine scratch or the like is sometimes generated at the groove for balloon engagement or in the periphery thereof, and it may become difficult to secure water tightness between the groove and the balloon. Thus, it is preferable to perform the removal of the balloon without using an instrument, if possible.

As a scheme of facilitating removal of a balloon, it is possible to use a technique of inflating the balloon by supplying water to the balloon to make the balloon to be easily pinched using fingers. In the conventional ultrasound endoscope, however, water is supplied from a water supply tank to the ultrasound endoscope by pressurizing the inside of the water supply tank using an air supply function provided in a light source device. Thus, it is difficult to supply the water to the balloon in a case where the ultrasound endoscope is not connected to the light source device. Thus, the balloon cannot be inflated at a location, such as a sink, which is separated from the light source device after using the ultrasound endoscope, which is inconvenient.

The present invention has been made in view of the foregoing, and an object of the invention is to provide an ultrasound endoscope capable of supplying water to a balloon provided in the ultrasound endoscope even when the ultrasound endoscope is not connected to a light source device, and provide an injection tool for injecting water into the balloon in the ultrasound endoscope.

### Solution to Problem

In order to solve the above described problem and achieve the object, an ultrasound endoscope according to the invention includes: an insertion portion configured to be inserted into a living body; a distal end rigid portion provided at a distal end of the insertion portion, the distal end rigid portion including: an ultrasound transmitting and receiving unit configured to transmit and receive ultrasound waves to and from the living body; a balloon surrounding the ultrasound transmitting and receiving unit; an engagement portion engaged with the balloon; and a water supply port for supplying water to the balloon; a balloon water supply channel leading to the water supply port to supply water toward the water supply port; a connector portion for connecting the ultrasound endoscope to a light source device; an operating unit for operating the ultrasound endoscope; a cable for connecting the connector portion to the operating unit; a water supply pipe provided in the cable; a water supply cylinder provided at the operating unit, one end of the balloon water supply channel and one end of the water supply pipe being opened to the water supply cylinder; an injection port provided on a flow path extending from the water supply pipe to the water supply port through the water supply cylinder and the balloon water supply channel; and an injection means for injecting water into the injection port.

In the ultrasound endoscope according to the invention, the injection port is provided in the balloon water supply channel. The injection means is a duct having one end leading to the balloon water supply channel via the injection port and having the other end to which a syringe for injecting water or supplying air to the duct is attachable.

The ultrasound endoscope according to the invention further includes a check valve provided closer to the water supply cylinder than the injection port in the balloon water supply channel to prevent flow from the injection port toward the water supply cylinder.

In the ultrasound endoscope according to the invention, the injection port is an open end of the balloon water supply channel that is opened to the water supply cylinder. The injection means is an injection tool configured to be inserted into the water supply cylinder. The injection tool includes therein a flow path having one end leading to the open end and having the other end to which a syringe for injecting water or supplying air to the flow path is attachable.

In the ultrasound endoscope according to the invention, the injection tool is configured to cover the water supply cylinder. The one end of the flow path is positioned with respect to the open end of the balloon water supply channel when the water supply cylinder is covered by the injection tool.

In the ultrasound endoscope according to the invention, the injection port is provided in the water supply pipe. The injection means is a duct having one end leading to the water supply pipe via the injection port and having the other end to which a syringe for injecting water or supplying air to the duct is attachable.

The ultrasound endoscope according to the invention further includes a check valve provided closer to the connector portion than the injection port in the water supply pipe to prevent flow from the injection port toward the connector portion.

An injection tool according to the invention is used in an ultrasound endoscope. The ultrasound endoscope includes: an ultrasound transmitting and receiving unit configured to transmit and receive ultrasound waves to and from a living body; a balloon surrounding the ultrasound transmitting and receiving unit; a balloon water supply channel for supplying water to the balloon; and a water supply cylinder to which one end of the balloon water supply channel is opened. The injection tool includes: an insertion portion that is insertable into the water supply cylinder; and a flow path having one end leading to an open end of the balloon water supply channel in the water supply cylinder and having the other end to which a syringe for injecting water or supplying air through the other end is attachable.

The injection tool according to the invention further includes a lid portion configured to cover the water supply cylinder. The one end of the flow path is positioned with respect to the open end when the water supply cylinder is covered by the lid portion. Advantageous Effects of Invention

According to the present invention, an injection port is provided on a flow path extending from a water supply pipe to a water supply port through a water supply cylinder and a balloon water supply channel, and water is injected into the injection port by an injection means. With this structure, even when the ultrasound endoscope is not connected to the light source device, it is possible to supply water to the balloon provided in the ultrasound endoscope.

### Brief Description of Drawings

FIG. 1 is a schematic view illustrating an ultrasound endoscopic system which includes an ultrasound endoscope according to a first embodiment of the present invention.
FIG. 2 is a partial cross-sectional view illustrating a configuration of the ultrasound endoscope according to the first embodiment of the present invention.
FIG. 3 is a partial cross-sectional view illustrating a configuration of an ultrasound endoscope according to a second embodiment of the present invention.
FIG. 4 is a partial cross-sectional view illustrating a configuration of an ultrasound endoscope according to a third embodiment of the present invention.
FIG. 5 is a cross-sectional view of a case where a plug portion is viewed from a direction indicated by arrows A illustrated in FIG. 4.
FIG. 6 is a partial cross-sectional view illustrating a configuration of an ultrasound endoscope according to a fourth embodiment of the present invention.
FIG. 7 is a schematic view illustrating a part of an ultrasound endoscope according to a fifth embodiment of the present invention.

### Description of Embodiments

Hereinafter, an ultrasound endoscope and an injection tool according to some embodiments of the present invention will be described with reference to the drawings. The present invention is not limited to these embodiments. The same reference signs are used to designate the same elements throughout the drawings. The drawings are schematic, and a relation between a thickness and a width of each member, each ratio of the members, and the like are different from the actual ones. The drawings may include some parts that have different dimensional relationships and ratios among the drawings.

### (First Embodiment)

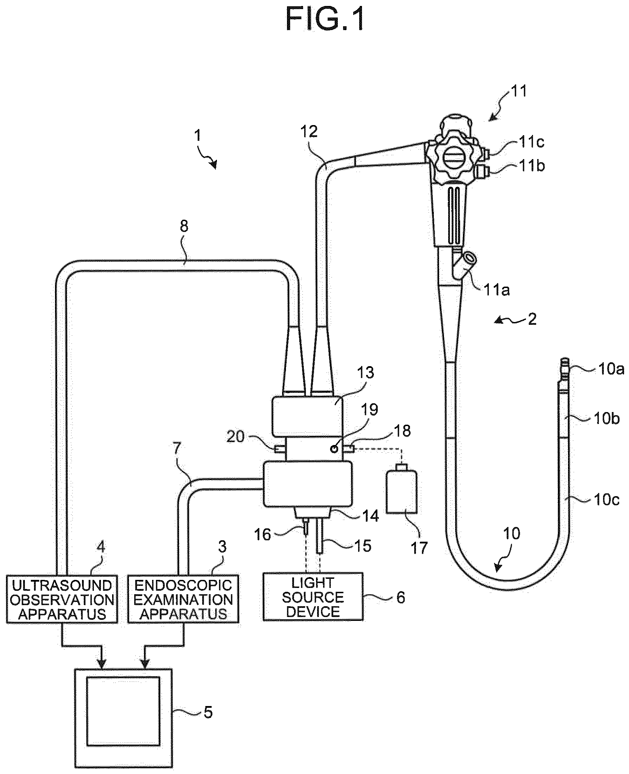

FIG. 1 is a schematic view illustrating an ultrasound endoscopic system which includes an ultrasound endoscope according to a first embodiment of the present invention. An ultrasound endoscopic system 1 illustrated in FIG. 1 includes an ultrasound endoscope 2, an endoscopic examination apparatus 3, an ultrasound observation apparatus 4, a display device 5, a light source device 6, a video cable 7 which connects the ultrasound endoscope 2 and the endoscopic examination apparatus 3, and an ultrasound cable 8 which connects the ultrasound endoscope 2 and the ultrasound observation apparatus 4.

The ultrasound endoscope 2 is obtained by combining an ultrasound transmitting and receiving unit with an endoscopic examination unit. The endoscopic examination unit includes an examination optical system including a lens, and an image sensor. The ultrasound endoscope 2 has an endoscopic examination function and an ultrasound observation function. The endoscopic examination apparatus 3 controls the endoscopic examination function, and processes an output signal output from the ultrasound endoscope 2 through endoscopic examination. The ultrasound observation apparatus 4 controls the ultrasound observation function, and processes an output signal output from the ultrasound endoscope 2 through ultrasound observation. The display device 5 acquires signals output from the endoscopic examination apparatus 3 and the ultrasound observation apparatus 4, and suitably displays at least one of an endoscopic image and an ultrasound tomogram. The light source device 6 includes therein a light source for generating illumination light to perform the endoscopic examination, and an air supply pump configured to supply air to the ultrasound endoscope 2.

The ultrasound endoscope 2 includes: an insertion portion 10 which is inserted into a living body, transmits an ultrasound signal inside the body, and receives the ultrasound signal reflected inside the body; an operating unit 11 which is continuously provided at a proximal end side of the insertion portion 10; and a universal cord 12 which extends from a side portion of the operating unit 11.

The insertion portion 10 is configured by continuously providing a distal end rigid portion 10a formed by a rigid member, a bending portion 10b that is bendable, and a flexible pipe portion 10c in the order from the distal end side. A proximal end of the flexible pipe portion 10c is continuously provided at the distal end side of the operating unit 11. The endoscopic examination unit and the ultrasound transmitting and receiving unit described above are arranged in the distal end rigid portion 10a.

The operating unit 11 is provided with a treatment tool insertion port 11a configured to introduce a treatment tool such as a puncture needle and forceps inside the body. In addition, the operating unit 11 is provided with a dial or an operating button configured to allow a user to operate the ultrasound endoscope 2. Specific examples thereof may include an air supply and water supply button 11b used at the time of supplying air or water to the body, a suction button 11c used at the time of suctioning liquid from the inside of the body, and the like.

A connector portion 13 is provided in an end at a side different from the operating unit 11 side of the universal cord 12. The video cable 7 and the ultrasound cable 8 are connected to the connector portion 13. The ultrasound endoscope 2 and the endoscopic examination apparatus 3 are electrically connected via the video cable 7. The ultrasound endoscope 2 and the ultrasound observation apparatus 4 are electrically connected via the ultrasound cable 8.

The connector portion 13 is provided with an inserting portion 14 configured to connect the ultrasound endoscope 2 to the light source device 6. The inserting portion 14 is provided with a light guide 15 and an air supply connector 16. When the inserting portion 14 is inserted into an inserting port (not illustrated) provided in the light source device 6, the ultrasound endoscope 2 and the light source device 6 are electrically connected to each other, and the illumination light generated by the light source device 6 is guided to the ultrasound endoscope 2 via the light guide 15. Then, air is supplied to the ultrasound endoscope 2 via the air supply connector 16 by the air supply pump provided in the light source device 6.

In addition, the connector portion 13 is provided with a water supply connector 18 to which a water supply tank 17 is connected via a water supply tube (not illustrated) and a pressurization pipe 19 configured to pressurize the inside of the water supply tank 17. At the time of supplying water to the ultrasound endoscope 2, the inside of the water supply tank 17 is pressurized by the air supply pump provided in the light source device 6, via the air supply connector 16 and the pressurization pipe 19. Accordingly, water inside the water supply tank 17 is supplied to the ultrasound endoscope 2 via the water supply connector 18.

Further, the connector portion 13 is provided with a suction ferrule 20 to which a suction device (not illustrated), which is configured to suction liquid or the like from the inside of the body by the ultrasound endoscope 2, is connected.

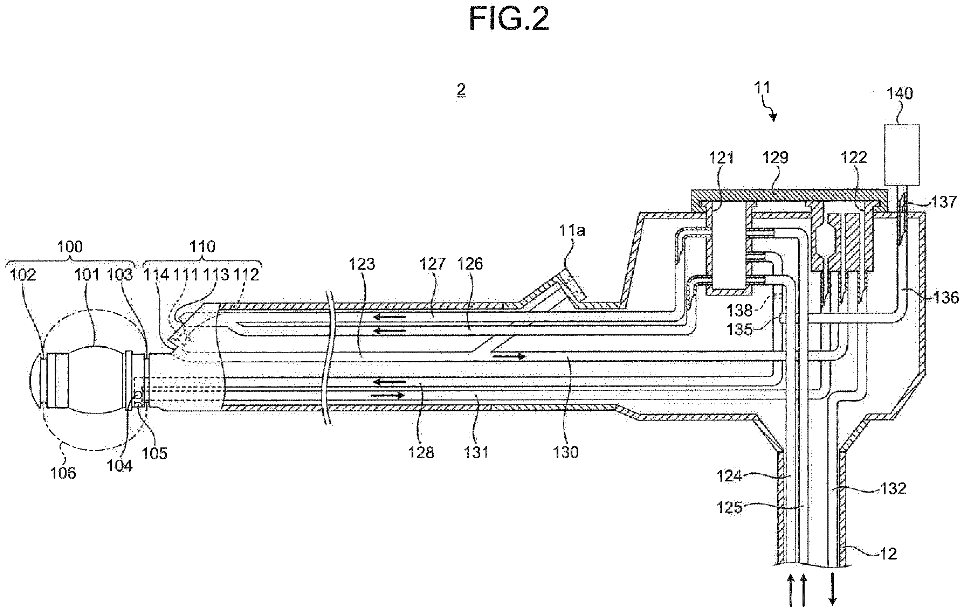

FIG. 2 is a partial cross-sectional view of the ultrasound endoscope 2. The distal end rigid portion 10a positioned at the distal end side of the insertion portion 10 includes an ultrasound transducer 100 to observe an affected tissue (an organ or the like as an examination target using an ultrasound wave, and an endoscopic examination unit 110 to optically examine a surface of the organ or the like.

The ultrasound transducer 100 may be any one of a radial transducer, a convex transducer, and a linear transducer. FIG. 2 illustrates the radial transducer as an example. The ultrasound transducer 100 includes an ultrasound transmitting and receiving unit 101 which transmits an ultrasound wave and receives the ultrasound wave (ultrasound echo) reflected by the examination target. Groove portions (engagement portions) 102 and 103 engaged with a balloon 106 which covers the ultrasound transmitting and receiving unit 101, are provided at a distal end side and a proximal end side of the ultrasound transmitting and receiving unit 101. The balloon 106 has a structure in which O-rings are provided at apertures at both ends of a flexible film (rubber film) formed in a tubular shape, and these O-rings are fitted and fixed to the groove portions 102 and 103, respectively. In addition, a balloon water supply port 104 configured to supply water to the balloon 106 and a balloon suction port 105 configured to suction water from the inside of the balloon 106 are provided in a region between the two groove portions 102 and 103.

Further, the groove for balloon engagement may be provided only at the proximal end side of the ultrasound transmitting and receiving unit 101 (only the groove portion 103 in FIG. 2). In this case, an O-ring provided in an aperture of a bag-shaped balloon is fitted into the groove at the proximal end side.

The endoscopic examination unit 110 includes a light-emitting element (not illustrated) to generate illumination light, an illumination lens (not illustrated) to guide the illumination light into the examination target (the surface of the organ or the like), an objective lens 111 to collect reflection light reflected by the surface of the organ or the like, an image sensor 112 which receives light collected by the objective lens 111 and converts the received light into an electrical signal, an air supply and water supply port 113 which serves as an outlet of air or water configured to clean the objective lens 111 and the like during endoscopic examination, and a suction and forceps port 114 which serves as not only a suction port to suction liquid or the like from the inside of the body but also an outlet of the treatment tool, such as the puncture needle and the forceps, to the inside of the body.

The operating unit 11 is also provided with an air supply and water supply cylinder 121 which switches a flow path of air or water to be supplied inside the insertion portion 10 and a suction cylinder 122 which switches a flow path of liquid or the like to be suctioned from the inside of the insertion portion 10, in addition to the above-described treatment tool insertion port 11a.

A treatment tool channel 123 communicating with the suction and forceps port 114 is provided inside the insertion portion 10, and the treatment tool insertion port 11a is an insertion port for the treatment tool into the treatment tool channel 123.

A water supply pipe 124 which is housed in the universal cord 12 and distributes the water supplied via the water supply connector 18, an air supply pipe 125 which is housed in the universal cord 12 and distributes the air supplied via the air supply connector 16, a water supply channel 126 and an air supply channel 127 communicating with the air supply and water supply port 113, and a balloon water supply channel 128 communicating with the balloon water supply port 104 have their ends opened to the air supply and water supply cylinder 121.

The air supply and water supply button 11b (see FIG. 1) is fitted into the air supply and water supply cylinder 121 during observation of the inside of the body. The air supply and water supply button 11b is a button switch for switching between a mode in which the water supply pipe 124 and water supply channel 126 communicate with each other to supply water from the air supply and water supply port 113, a mode in which the air supply pipe 125 and the air supply channel 127 communicate with each other to supply air from the air supply and water supply port 113, and a mode in which the water supply pipe 124 and the balloon water supply channel 128 communicate with each other to supply water from the balloon water supply port 104 to the balloon 106.

A suction channel 130 branched from the treatment tool channel 123, a balloon suction channel 131 communicating with the balloon suction port 105, and a suction pipe 132 housed in the universal cord 12 have their ends opened to the suction cylinder 122.

The suction button 11c (see FIG. 1) is fitted into the suction cylinder 122 during observation of the inside of the body. The suction button 11c is a button switch for switching between a mode in which the suction channel 130 and the suction pipe 132 communicate with each other to suction liquid or the like through the suction and forceps port 114 and a mode in which the balloon suction channel 131 and the suction pipe 132 communicate with each other to suction water in the balloon 106 through the balloon suction port 105.

In addition, the air supply and water supply cylinder 121 and the suction cylinder 122 is covered by a duct plug 129 at the time of cleaning the ultrasound endoscope 2.

An injection port 135 configured to directly inject water into the balloon water supply channel 128 is provided at a part of the balloon water supply channel 128. A duct 136 is connected to the injection port 135. An end of the duct 136 at the opposite side to the injection port 135 is opened to the outside of a casing of the operating unit 11, and a port 137 to which a water injection syringe 140 can be mounted is provided at this aperture. Further, the port 137 is covered by a dedicated lid (not illustrated) to be sealed during observation of the inside of the body. Alternatively, a check valve to prevent a back flow to the port 137 may be provided in the middle of the duct 136. In addition, a check valve 138 to prevent flow toward the air supply and water supply cylinder 121 from the injection port 135 is provided at a more upstream side (the air supply and water supply cylinder 121 side) as compared to the injection port 135 of the balloon water supply channel 128.

At the time of observing the inside of the body using the ultrasound endoscopic system 1, the O-rings provided at both the ends of the balloon 106 are fitted and fixed to the groove portions 102 and 103, respectively, and the insertion portion 10 is inserted into the body. Then, the water that has been supplied via the water supply pipe 124 and the balloon water supply channel 128 is supplied through the balloon water supply port 104 to fill the inside of the balloon 106 with the water, and the balloon 106 brought into contact with the examination target to perform the ultrasound observation. In addition, the examination target is illuminated via the illumination lens to optically examine the inside of the body.

When the observation of the inside of the body is ended, the water inside the balloon 106 is suctioned through the balloon suction port 105, and the water is discharged via the balloon suction channel 131 and the suction pipe 132. Then, the insertion portion 10 is pulled out from the inside of the body in the state where the balloon 106 is deflated. Thereafter, the ultrasound endoscope 2 is taken out of the light source device 6.

At the time of cleaning the ultrasound endoscope 2, the water injection syringe 140 is attached to the port 137. The balloon 106 is inflated by supplying water from the water injection syringe 140 to the balloon 106 via the duct 136 and the balloon water supply channel 128. Accordingly, the balloon 106 can be easily taken out of the groove portions 102 and 103.

As described above, the injection port 135 is provided in the balloon water supply channel 128 so that the water is directly injected into the balloon water supply channel 128 via the duct 136 and the injection port 135 using the water injection syringe 140 according to the first embodiment of the present invention. Thus, it is possible to supply the water to the balloon 106 to inflate the balloon 106 even when the ultrasound endoscope 2 is not connected to the light source device 6. Accordingly, it is possible to efficiently perform the cleaning of the ultrasound endoscope 2.

As a modified example of the above-described first embodiment, the balloon 106 may be inflated by supplying air instead of injecting water from the water injection syringe 140. Also in this case, it is possible to easily take out the balloon 106 by inflating the balloon 106. In the case of supplying the air, an air supply means, such as a syringe for air supply, may be attached to the port 137.

### (Second Embodiment)

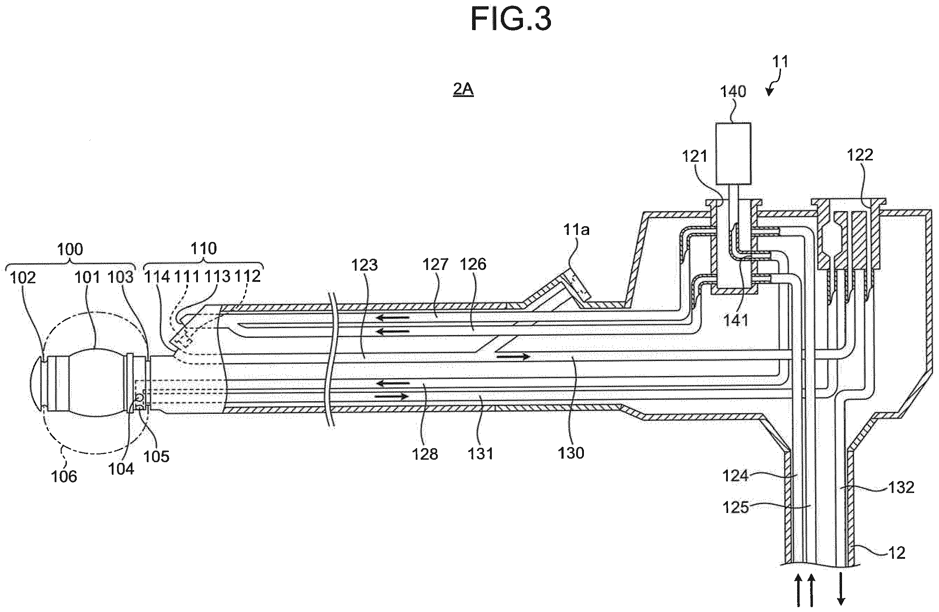

Next, a second embodiment of the present invention will be described. FIG. 3 is a partial cross-sectional view illustrating a configuration of an ultrasound endoscope according to the second embodiment of the present invention. Configurations of the respective parts of the ultrasound endoscope are the same as those in the first embodiment, except for a connection structure of a water injection syringe 140 with respect to a balloon water supply channel 128.

As illustrated in FIG. 3, the water injection syringe 140 is connected to an open end 141 of a balloon water supply channel 128 opened to an air supply and water supply cylinder 121 in an ultrasound endoscope 2A according to the second embodiment, instead of providing an injection port 135 in the middle of the balloon water supply channel 128 and connecting a duct 136 thereto (see FIG. 2).

In this manner, even when the water is directly injected to the balloon water supply channel 128 from the water injection syringe 140, it is possible to supply water to the balloon 106 to inflate the balloon.

Further, in the second embodiment, the balloon 106 may also be inflated by supplying air instead of injecting water from the water injection syringe 140.

### (Third Embodiment)

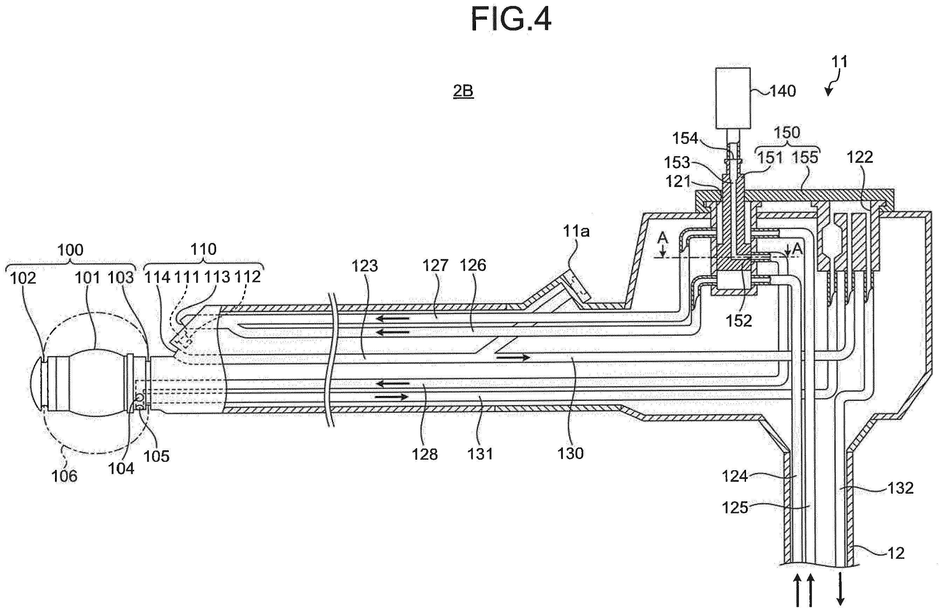

Next, a third embodiment of the present invention will be described. FIG. 4 is a partial cross-sectional view illustrating a configuration of an ultrasound endoscope according to the third embodiment of the present invention. Configurations of the respective parts of the ultrasound endoscope are the same as those in the first embodiment, except for a connection structure of a water injection syringe 140 with respect to a balloon water supply channel 128.

As illustrated in FIG. 4, an ultrasound endoscope 2B according to the third embodiment includes a duct plug 150 serving as an injection tool. The duct plug 150 includes a plug portion 151 inserted into an air supply and water supply cylinder 121 and a lid portion 155 which can cover the air supply and water supply cylinder 121 and a suction cylinder 122.

A flow path 153, which communicates with an open end 152 of a balloon water supply channel 128 at one end and is opened to the outside of an operating unit 11 at the other end, is formed inside the plug portion 151. A port 154 to which a water injection syringe 140 can be connected is provided at the other end of the flow path 153. The flow path 153 is positioned such that one open end of the flow path 153 matches the open end 152 of the balloon water supply channel 128 at the time of covering the air supply and water supply cylinder 121 and the suction cylinder 122 with the lid portion 155.

At the time of cleaning the ultrasound endoscope 2, the plug portion 151 is inserted into the air supply and water supply cylinder 121, the lid portion 155 is placed to cover the air supply and water supply cylinder 121 and the suction cylinder 122, and the water injection syringe 140 is attached to the port 154. It is possible to supply water to a balloon 106 via the flow path 153 and the balloon water supply channel 128 by performing the water injection from the water injection syringe 140, and to easily take the balloon 106 out of groove portions 102 and 103 by inflating the balloon 106.

Further, in the third embodiment, the balloon 106 may also be inflated by supplying air instead of injecting water from the water injection syringe 140.

### (Modified Example)

A modified example of the third embodiment of the present invention will be described. As illustrated in FIG. 4, a bottom surface of the plug portion 151 may be positioned to be positioned at an upper side than each open end of the water supply pipe 124 and water supply channel 126 in the air supply and water supply cylinder 121. In this case, a manual injection means such as a water injection tube is connected to the water supply pipe 124, and the water supplied to the water supply pipe 124 from the manual injection means can be supplied to the water supply channel 126 via the air supply and water supply cylinder 121. Accordingly, it is also possible to perform manual cleaning (manually cleaning without using a cleaning device) of the water supply channel 126.

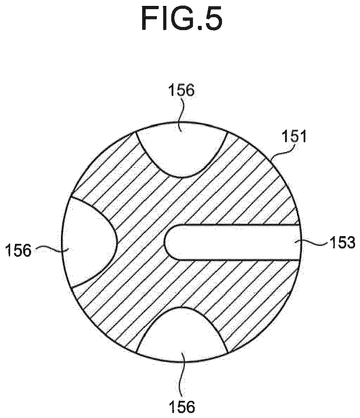

In addition, FIG. 4 illustrates a state where the inside of the air supply and water supply cylinder 121 is closed by the plug portion 151, but a clearance may be provided in the plug portion 151 such that water flows inside the air supply and water supply cylinder 121. FIG. 5 illustrates a cross section of a case where the plug portion 151 is viewed from a direction indicated by arrows A illustrated in FIG. 4. As illustrated in FIG. 5, it is also possible to supply water, which has been supplied to the water supply pipe 124 from the manual injection means, to the air supply channel 127 via the air supply and water supply cylinder 121 by providing a plurality of clearances 156 in the plug portion 151. Accordingly, it is possible to perform manual cleaning of the air supply channel 127 in addition to the water supply channel 126.

### (Fourth Embodiment)

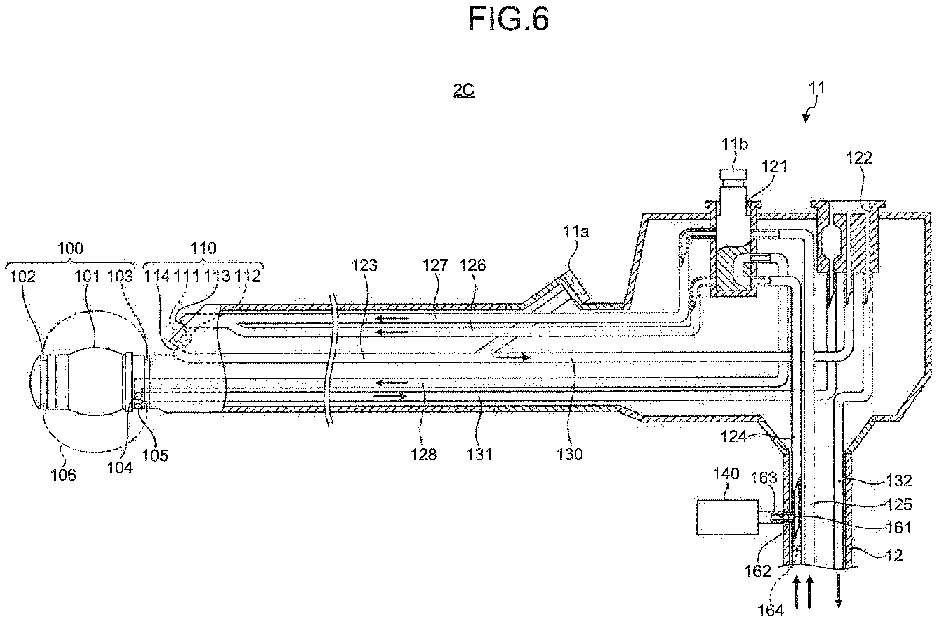

Next, a fourth embodiment of the present invention will be described. FIG. 6 is a partial cross-sectional view illustrating a configuration of an ultrasound endoscope according to the fourth embodiment of the present invention. Configurations of the respective parts of the ultrasound endoscope are the same as those in the first embodiment, except for a structure for performing water injection from a water injection syringe 140 with respect to a balloon water supply channel 128.

As illustrated in FIG. 6, an injection port 161 configured to directly inject water into a water supply pipe 124 is provided at a part of the water supply pipe 124 in an ultrasound endoscope 2C according to the fourth embodiment. A duct 162 is connected to the injection port 161. An end of the duct 162 at the opposite side to the injection port 161 is opened to the outside of a proximal end of a universal cord 12, and a port 163 to which a water injection syringe 140 can be mounted is provided at this aperture. Further, the port 163 is covered by a dedicated lid (not illustrated) to be sealed during observation of the inside of the body. Alternatively, a check valve to prevent a back flow in a direction to the port 163 may be provided in the middle of the duct 162. In addition, a check valve 164 to prevent flow toward a connector portion 13 side from the injection port 161 is provided at a more upstream side (the connector portion 13 side) as compared to the injection port 161 of the water supply pipe 124. The air supply and water supply button 11b is inserted into the air supply and water supply cylinder 121.

At the time of cleaning the ultrasound endoscope 2, the air supply and water supply button 11b is switched to the mode in which the water supply pipe 124 and the balloon water supply channel 128 communicate with each other to supply water to a balloon 106. Then, the water injection syringe 140 is attached to the port 163. It is possible to supply water to the balloon 106 via the water supply pipe 124, the flow path inside the air supply and water supply button 11b, and the balloon water supply channel 128 by injecting the water injection from the water injection syringe 140 into the duct 162, and to easily take the balloon 106 out of the groove portions 102 and 103 by inflating the balloon 106.

Further, in the fourth embodiment, the balloon 106 may also be inflated by supplying air instead of injecting water from the water injection syringe 140.

### (Fifth Embodiment)

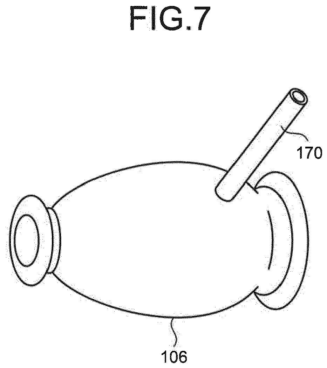

Next, a fifth embodiment of the present invention will be described. FIG. 7 is a schematic view illustrating a part of an ultrasound endoscope according to the fifth embodiment of the present invention.

As illustrated in FIG. 7, a port 170 to which a water injection syringe 140 can be mounted may be directly provided to a balloon 106. This port 170 is attached outside an ultrasound scanning region so as not to be reflected in an ultrasound image. Further, the port 170 is attached to a position which is not included in a field of vision of an objective lens 111 (see FIG. 2) of an endoscopic examination unit 110 so as not to be reflected in an endoscopic examination image.

At the time of cleaning the ultrasound endoscope, the water injection syringe 140 is attached to the port 170, and water is injected inside the balloon 106. Accordingly, it is possible to easily take the balloon 106 out of groove portions 102 and 103 by inflating the balloon 106.

Further, in the fifth embodiment, the balloon 106 may also be inflated by supplying air instead of injecting water from the water injection syringe 140.

The present invention described as above is not limited to the first to fifth embodiments, and various modifications can be made based on specification or the like. For example, modifications can be formed by excluding several elements from the entire elements in the first to fifth embodiments. From the above description, it is obvious that other various embodiments can be made within a scope of the present invention.

### Industrial Applicability

As described above, the ultrasound endoscope according to the present invention and the injection tool for injecting water into the balloon in the ultrasound endoscope are useful for supplying water to the balloon attached to the ultrasound endoscope even when the ultrasound endoscope is not connected to the light source device.

### Reference Signs List

1 ULTRASOUND ENDOSCOPIC SYSTEM
2, 2A, 2B, 2C ULTRASOUND ENDOSCOPE
3 ENDOSCOPIC EXAMINATION APPARATUS
4 ULTRASOUND OBSERVATION APPARATUS
5 DISPLAY DEVICE
6 LIGHT SOURCE DEVICE
7 VIDEO CABLE
8 ULTRASOUND CABLE
10 INSERTION PORTION
11 OPERATING UNIT
11a TREATMENT TOOL INSERTION PORT
11b AIR SUPPLY AND WATER SUPPLY BUTTON
11c SUCTION BUTTON
12 UNIVERSAL CORD
13 CONNECTOR UNIT
14 INSERTING PORTION
15 LIGHT GUIDE
16 AIR SUPPLY CONNECTOR
17 WATER SUPPLY TANK
18 WATER SUPPLY CONNECTOR
19 PRESSURIZATION PIPE
20 SUCTION FERRULE
100 ULTRASOUND TRANSDUCER
110 ENDOSCOPIC EXAMINATION UNIT
101 ULTRASOUND TRANSMITTING AND RECEIVING UNIT
106 BALLOON
102, 103 GROOVE PORTION
104 BALLOON WATER SUPPLY PORT
105 BALLOON SUCTION PORT
111 OBJECTIVE LENS
112 IMAGE SENSOR
113 AIR SUPPLY AND WATER SUPPLY PORT
114 SUCTION AND FORCEPS PORT
121 AIR SUPPLY AND WATER SUPPLY CYLINDER
122 SUCTION CYLINDER
123 TREATMENT TOOL CHANNEL
124 WATER SUPPLY PIPE
125 AIR SUPPLY PIPE
126 WATER SUPPLY CHANNEL
127 AIR SUPPLY CHANNEL
128 BALLOON WATER SUPPLY CHANNEL
129, 150 DUCT PLUG
130 SUCTION CHANNEL
131 BALLOON SUCTION CHANNEL
132 SUCTION PIPE
135, 161 INJECTION PORT
136, 162 DUCT
137, 154, 163, 170 PORT
138, 164 CHECK VALVE
140 WATER INJECTION SYRINGE
141, 152 OPEN END
151 PLUG PORTION
153 FLOW PATH
155 LID PORTION
156 CLEARANCE

## Claims

1. An ultrasound endoscope comprising:
an insertion portion configured to be inserted into a living body;
a distal end rigid portion provided at a distal end of the insertion portion, the distal end rigid portion including: an ultrasound transmitting and receiving unit configured to transmit and receive ultrasound waves to and from the living body; a balloon surrounding the ultrasound transmitting and receiving unit; an engagement portion engaged with the balloon; and a water supply port for supplying water to the balloon;
a balloon water supply channel leading to the water supply port to supply water toward the water supply port;
a connector portion for connecting the ultrasound endoscope to a light source device;
an operating unit for operating the ultrasound endoscope;
a cable for connecting the connector portion to the operating unit;
a water supply pipe provided in the cable;
a water supply cylinder provided at the operating unit, one end of the balloon water supply channel and one end of the water supply pipe being opened to the water supply cylinder;
an injection port provided on a flow path extending from the water supply pipe to the water supply port through the water supply cylinder and the balloon water supply channel; and
an injection means for injecting water into the injection port.

2. The ultrasound endoscope according to claim 1, wherein
the injection port is provided in the balloon water supply channel, and
the injection means is a duct having one end leading to the balloon water supply channel via the injection port and having the other end to which a syringe for injecting water or supplying air to the duct is attachable.

3. The ultrasound endoscope according to claim 2, further comprising a check valve provided closer to the water supply cylinder than the injection port in the balloon water supply channel to prevent flow from the injection port toward the water supply cylinder.

4. The ultrasound endoscope according to claim 1, wherein
the injection port is an open end of the balloon water supply channel that is opened to the water supply cylinder,
the injection means is an injection tool configured to be inserted into the water supply cylinder, and
the injection tool includes therein a flow path having one end leading to the open end and having the other end to which a syringe for injecting water or supplying air to the flow path is attachable.

5. The ultrasound endoscope according to claim 4, wherein
the injection tool is configured to cover the water supply cylinder, and
the one end of the flow path is positioned with respect to the open end of the balloon water supply channel when the water supply cylinder is covered by the injection tool.

6. The ultrasound endoscope according to claim 1, wherein
the injection port is provided in the water supply pipe, and
the injection means is a duct having one end leading to the water supply pipe via the injection port and having the other end to which a syringe for injecting water or supplying air to the duct is attachable.

7. The ultrasound endoscope according to claim 6, further comprising a check valve provided closer to the connector portion than the injection port in the water supply pipe to prevent flow from the injection port toward the connector portion.

8. An injection tool used in an ultrasound endoscope, the ultrasound endoscope comprising: an ultrasound transmitting and receiving unit configured to transmit and receive ultrasound waves to and from a living body; a balloon surrounding the ultrasound transmitting and receiving unit; a balloon water supply channel for supplying water to the balloon; and a water supply cylinder to which one end of the balloon water supply channel is opened, the injection tool comprising:
an insertion portion that is insertable into the water supply cylinder; and
a flow path having one end leading to an open end of the balloon water supply channel in the water supply cylinder and having the other end to which a syringe for injecting water or supplying air through the other end is attachable.

9. The injection tool according to claim 8, further comprising a lid portion configured to cover the water supply cylinder,
wherein the one end of the flow path is positioned with respect to the open end when the water supply cylinder is covered by the lid portion.
